# EUROPÄISCHE PATENTSCHRIFT

(11) **EP 1 467 781 B1**
(45) Veröffentlichungstag und Bekanntmachung des Hinweises auf die Patenterteilung: **29.08.2007**
(21) Anmeldenummer: 03702507.9
(22) Anmeldetag: 23.01.2003
(51) Int. Cl.: A61M 5/00, A61M 27/00, A61C 19/04, A61C 3/03, A61C 1/08, A61C 17/02, A61M 3/00, A61M 1/00, A61B 17/32

(54) **Dentales Behandlungsgeràt**
Dental therapeutic device
Appareil de traitement dentaire

(30) Priorität: 23.01.2002 DE 10202378
(43) Veröffentlichungstag der Anmeldung: 20.10.2004
(73) Patentinhaber: Dürr Dental GmbH & Co. KG, 74321 Bietigheim-Bissingen (DE); Hahn, Rainer, Dr., 72074 Tübingen (DE)
(72) Erfinder: HAHN, Rainer, 72074 Tübingen (DE); PRAGER, Ulrich, 74232 Abstatt (DE)
(74) Vertreter: Ostertag, Reinhard
(86) Internationale Anmeldenummer: PCT/EP2003/000662
(87) Internationale Veröffentlichungsnummer: WO 2003/061734

(56) Entgegenhaltungen:
- EP-A- 0 636 345
- WO-A-01/37922
- WO-A-01/97700
- DE-A- 4 331 289
- DE-C- 3 739 563
- GB-A- 2 125 487
- US-A- 4 817 599
- US-A- 5 180 371
- US-A- 5 542 918
- US-A- 5 558 646
- US-A- 6 159 161

## Beschreibung

Die Erfindung betrifft ein dentales Behandlungsgerät zum Infiltrieren und/oder Spülen von Gewebe oder gewebebegrenzten Hohlräumen, insbesondere von Zahngewebe oder Zahngewebe-begrenzten Hohlräumen, mit einer Behandlungsflüssigkeit mit
a) einem Vorratsbehälter für die Behandlungsflüssigkeit;
b) einer Kanüle zum Einbringen der Behandlungsflüssigkeit in das Gewebe bzw. die Hohlräume;
c) einer Pumpe, welche der Kanüle die Behandlungsflüssigkeit aus dem Vorratsbehälter zuführt;
d) einer Pumpe, welche über die Kanüle Behandlungsflüssigkeit aus dem Gewebe absaugt;
e) bei der vorratsbehälter, Kanüle und Pumpen zu einer handstückigartigen Einheit zusammengefaßt sind;
f) und bei der die Pumpe, welche Behandlungsflüssigkeit der Kanüle zuführt, und die Pumpe, welche die Behandlungsflüssigkeit über die Kanüle absaugt, durch eine einzige Pumpe implementiert sind, deren Arbeitsrichtung umumkehrbar ist.

In der US-A 6 159 161 ist ein solches dentales Behandlungsgerät gezeigt, welches dazu dient, Flüssigkeit über eine Kanüle abzugeben. Das Gerät ist insgesamt handstückartig ausgebildet und umfaßt einen Vorratsbehälter. Eine Pumpe, die über ein Getriebe von einem Motor angetrieben wird, saugt aus dem Vorratsbehälter an und ist auslaßseitig mit der Kanüle verbunden. Das Handstück dient in erster Linie zum Abgeben eines Anästhetikums, es kann durch Umpolen des auf die Pumpe arbeitenden Motors aber auch so betrieben werden, daß über die Kanüle Flüssigkeit aus dem Gewebe abgesaugt wird.

In der US-A-5 180 371 ist eine Spritze gezeigt, die ebenfalls eine durch einen Motor angetriebene Pumpe aufweist, die Flüssigkeit, insbesondere ein Anästhetikum, einer Kanüle zuführt und bei Umpolung Flüssigkeit über die Kanüle ansaugen kann.

Ein weiteres Behandlungsgerät ist aus der DE 197 14 167 A1, insbesondere aus der dortigen Figur 4, bekannt. In dieser Druckschrift ist auch im einzelnen angegeben, welche Behandlungsflüssigkeiten in Frage kommen und welcher Zweck mit der Infiltration und/oder Spülung des Gewebes verbunden ist.

Die DE 37 39 563 C1 offenbart eine Infusionsspritze, welche eine normale Kolbenspritze und einen Servoantrieb für deren Kolben aufweist. Der Servoantrieb umfasst einen auf eine Gewindespindel arbeitenden Elektromotor und eine auf der Gewindespindel laufende Antriebsmutter, die mit dem Kolben der Spritze gekoppelt ist. Antriebseinheit und Spritze sind parallel übereinander gesetzt.

Die GB 2 125 487 A offenbart eine Doppelspritze zur Verwendung bei Operationen, wobei eine der Spritzen Behandlungsflüssigkeit an einen Operationsort abgeben kann und die andere von dort störende Flüssigkeit absaugen kann. Die beiden Spritzen werden durch getrennte Antrieb im Gegentakt bewegt. Die gesamte Einheit braucht verhältnismäßig viel Platz und ist verhältnismäßig schwer, weshalb sie an einem Ständer oder dgl. befestigt wird.

Die US 4 817 599 A offenbart eine spezielle Spritze zur Verwendung bei Staroperationen. Mit ihr ist es möglich, eine Behandlungsflüssigkeit zum Operationsort zu führen und von dort verbrauchte und Gewebereste enthaltende Flüssigkeit abzusaugen. Ein vorratsbehälter für Behandlungsflüssigkeit ist mit der Spritze über einen Schlauch verbunden.

Bei dem in der DE 197 14 167 A1 beschriebenen Behandlungsgerät wird ein Handstück eingesetzt, welches im wesentlichen nur die Kanüle und bestimmte Sensoren und Steuerelemente umfaßt, die dort deshalb erforderlich sind, weil dieses Behandlungsgerät auch aus Diagnosegerät eingesetzt werden soll. Die Pumpen und vorratsbehälter sowie die anderen zum Betrieb erforderlichen Aggregate sind aus dem Handstück ausgelagert und mit diesem über ein Versorgungskabel verbunden. Das gesamte Behandlungsgerät nimmt auf diese Weise in der Zahnarztpraxis verhältnismäßig viel Platz ein und erfordert einen vergleichsweise großen Investitionsaufwand, da von in der Zahnarztpraxis bereits vorhanden Aggregaten kein Gebrauch gemacht wird.

Aufgabe der vorliegenden Erfindung ist es, ein Behandlungsgerät der eingangs genannten Art so auszugestalten, daß es einfach handhabbar und kostengünstig ist sowie wenig Platz benötigt.

Diese Aufgabe wird erfindungsgemäß dadurch gelöst, daß
g) die einzige Pumpe einen doppelt wirkenden, linear beweglichen Kolben umfaßt, der mit einem Endbereich an einen ersten Arbeitsraum angrenzt, welcher über ein Rückschlagventil mit dem Reservoir verbunden ist, und mit dem gegenüberliegenden Endbereich an einen zweiten Arbeitsraum angrenzt, der mit der Kanüle kommuniziert, wobei der erste Arbeitsraum mit dem zweiten Arbeitsraum über ein Strömungsweg kommuniziert, in welchem ein Rückschlagventil liegt, das eine Strömung der Behandlungsflüssigkeit nur vom ersten Arbeitsraum in den zweiten Arbeitsraum zuläßt.

Erfindungsgemäß wird also das Behandlungsgerät von einer Einheit gebildet, die in derselben Weise wie andere in einer Zahnarztpraxis gebräuchliche Handstücke gehandhabt werden kann, jedoch alle erforderlichen Komponenten in sich vereinigt und allenfalls auf Aggregate zurückgreift, die in einer Zahnarztpraxis regelmäßig anzufinden sind. Kosten und Raumbedarf sind auf diese Weise gegenüber dem Stande der Technik erheblich reduziert.

Diese Zusammenfassung beider Pumpenfunktionen in einer einzigen Pumpe verringert den für die Pumpen erforderlichen Raumbedarf, was bei der erfindungsgemäß Integration in ein Handstück besonders wichtig ist. Möglich wird diese Zusammenfassung der beiden Pumpenfunktionen deshalb, weil bei dem erfindungsgemäßen Behandlungsgerät immer nur eine der beiden Pumpfunktionen benötigt werden, so daß ein alternierender Betrieb der einzigen Pumpe mit sich ändernder Arbeitsrichtung möglich ist.

Bewegt sich der bei dem erfindungsgemäßen Gerät vorgesehene doppeltwirkende Kolben in einer Richtung, so wird einerseits aus dem Vorratsbehälter in den ersten Arbeitsraum Behandlungsflüssigkeit eingesaugt und andererseits Behandlungsflüssigkeit aus dem zweiten Arbeitsraum zur Kanüle hin ausgestoßen. Bei der in entgegengesetzter Richtung laufenden Bewegung des Kolbens wird die im ersten Arbeitsraum befindliche Behandlungsflüssigkeit in den zweiten Arbeitsraum verdrängt, in welchen außerdem über die Kanüle aus dem Gewebe bzw. dem Hohlraum Flüssigkeit eingesaugt wird. Bei letzterer handelt es sich um ein Gemisch aus Gewebsflüssigkeit, Speichel, Debris und Behandlungsflüssigkeit. Diese Bauweise kommt für die angestrebten Funktionen mit außerordentlich wenigen Baukomponenten aus, was wiederum dem Raumbedarf und den Kosten zugute kommt.

Der vom ersten Arbeitsraum zum zweiten Arbeitsraum fühende Strömungsweg kann eine axial durch den Kolben geführte Bohrung sein, was wiederum den Platzbedarf verringert.

Im allgemeinen wird aufgrund von Leckverlusten die in das Gewebe injizierte Behandlungsflüssigkeit nicht mehr in vollem Umfange zurückgesaugt; die zum Spülen und/oder Infiltrieren eingesetzte Behandlungsflüssigkeit wird vielmehr ständig aus dem Reservoir ergänzt. Hierdurch wird eine spülwirkung erzielt, da mit der überschießenden Behandlungsflüssigkeit die größte Menge an Debris ausgespült wird, bevor das Hauptvolumen der Flüssigkeit zurückgesaugt wird. Hierzu empfiehlt sich eine Ausgestaltung der Erfindung, bei welcher der Querschnitt des an den ersten Arbeitsraum angrenzenden Endbereichs des Kolbens kleiner ist als der Querschnitt des an den zweiten Arbeitsraum angrenzenden Endbereichs des Kolbens. Aufgrund des kleineren Querschnitts des ersten Endbereiches wird bei jedem Kolbenhub der entsprechenden Richtung aus dem Vorratsbehälter weniger Behandlungsflüssigkeit entnommen als gleichzeitig aus dem zweiten Arbeitsraum in Richtung zur Kanüle ausgestoßen wird. Beim Rückwärtshub des Kolbens wird dann das zurückgesaugte Volumen der Flüssigkeit durch dasjenige Volumen ergänzt, welches zuvor in den ersten Arbeitsraum aus dem Vorratsbehälter eingesaugt wurde.

Die Betriebsweise des Behandlungsgerätes, bei welcher die Behandlungsflüssigkeit im Takt sowohl in das Gewebe bzw. die angrenzenden Hohlräume injiziert als auch aus dem Gewebe bzw. den Hohlräumen wieder abgesaugt wird, eignet sich besonders zur gründlichen Infiltration. Die getaktete Bewegung erlaubt ein sehr effektives Spülen durch intensiven Austausch zwischen Gewebsflüssigkeit und Behandlungsflüssigkeit. Insbesondere werden die Wirkstoffe der Behandluhgsflüssigkeit gut in tiefe Gewebsabschnitte befördert und Debris einschließlich Bakterien werden gut von Grenzflächen abgelöst. Mit der Leckage wird ein Teil des Debris usgestoßen, ein anderer Teil der Gewebsflüssigkeit wird zurückgesaugt, mit Behandlungsflüssigkeit vermischt und wieder eingefördert. Im Ergebnis wird durch die Leckage eine gute Reinigung erzielt.

Bevor die getaktete Bewegung eingeleitet wird, sollte allerdings zunächst kurzzeitig eine reine Spülung stattfinden, mit der das meiste Debris ausgestoßen wird.

In vielen Fällen ist jedoch auch eine reine Spülung des Gewebes erwünscht, bei welcher ausschließlich frische Behandlungsflüssigkeit in das Gewebe eingebracht und keine Behandlungsflüssigkeit zurückgesaugt wird. Hierfür eignet sich diejenige Ausführungsform der Erfindung, bei welcher ein Steuerventil vorgesehen ist, welches in einer ersten Stellung den zweiten Arbeitsraum über einen in beiden Richtungen durchströmbaren Strömungsweg mit der Kanüle verbindet und in einer zweiten Stellung den zweiten Arbeitsraum über einen nur in Richtung zur Kanüle durchströmbaren Strömungsweg mit der Kanüle und mit einem weiteren, zum Reservoir führenden Strömungsweg verbindet, wobei in dem weiteren Strömungsweg ein Rückschlagventil liegt, das ausschließlich eine Strömung in Richtung zum zweiten Arbeitsraum zuläßt.

In dieser spül-Betriebsart wird also dasjenige Volumen Behandlungsflüssigkeit, welches in der ersten Betriebsart aus dem Gewebe zurückgesaugt wird, durch frische, aus dem Vorratsbehälter stammende Behandlungsflüssigkeit ersetzt.

Das Steuerventil kann einen in einer Bohrung linear verschiebbaren Schieber umfassen.

Zweckmäßig ist ferner, wenn der doppet wirkende Kolben von einem Betätigungskolben angetrieben ist, der an einer Seite durch eine Druckfeder beaufschlagt ist und an der gegenüberliegende Seite an einen Druckraum angrenzt, der seinerseits mit dem Ausgang eines Druckluft-Impulsgebers kommuniziert. Zum Betrieb dieses Behandlungsgerätes ist also nur eine Druckluftquelle erforderlich, wie sie in allen Zahnarztpraxen ohnehin zur verfügung steht. Eine gesondere Anschaffung ist diesbezüglich nicht nötig. Der Druckluft-Impulsgeber, der entsprechend seinem Namen mit einer bestimmten Wiederholfrequenz Druckluftimpulse erzeugt, ist also solcher im Handel erhältlich.

Zweckmäßig in diesem Zusammenhang ist weiter, wenn der Eingang des Druckluft-Impulsgebers über eine Standardkupplung mit einem Druckluft-Versorgungskabel für übliche zahnärztlich Handstücke verbindbar ist. Der Zahnarzt kann also das erfindungsgemäße, entsprechend dieser Ausführungsform ausgestaltete Behandlungsgerät einfach an denselben Versorgungsschlauch ankoppeln, über den er auch seine sonstigen Handstücke betreibt.

Beim dem Reservoir kann es sich eine lösbar angeordnete Spritze handeln, die einen leichtgängigen Spritzenkolben aufweist. Die Leichtgängigkeit des Spritzenkolbens ist deshalb erforderlich, weil er sich ausschließlich unter dem Einfluß des von der Pumpe erzeugte Vakuums bewegt, welches die Spritze leert. Auf den Spritzenkolben wird also kein externer Druck zum Entleeren der Spritze ausgeübt.

Bei der Spritze kann es sich um eine wiederverwertbare, aus autoklavierbarem Material bestehende Spritze, alternativ aber auch um eine Wegwerfspritze handeln. Im letzteren Falle ist es nicht erforderlich, daß die Wegwerfspritze eine Kolbenstange aufweist, da diese Spritze ja nur entleert wird und dies unter dem Einfluß der saugenden Pumpe erfolgt.

Ein Ausführungsbeispiel der Erfindung wird nachfolgend anhand der Zeichnung näher erläutert; es zeigen
- Figur 1:: einen Schnitt durch ein Ausführungsbeispiel eines dentalen Behandlungsgerätes; und
- Figur 2:: eine Ausschnittvergrößerung aus Figur 1.

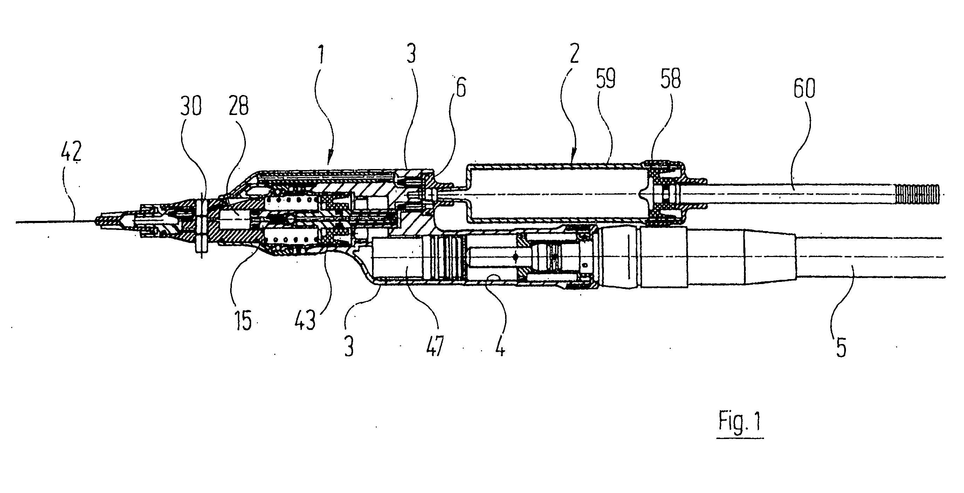

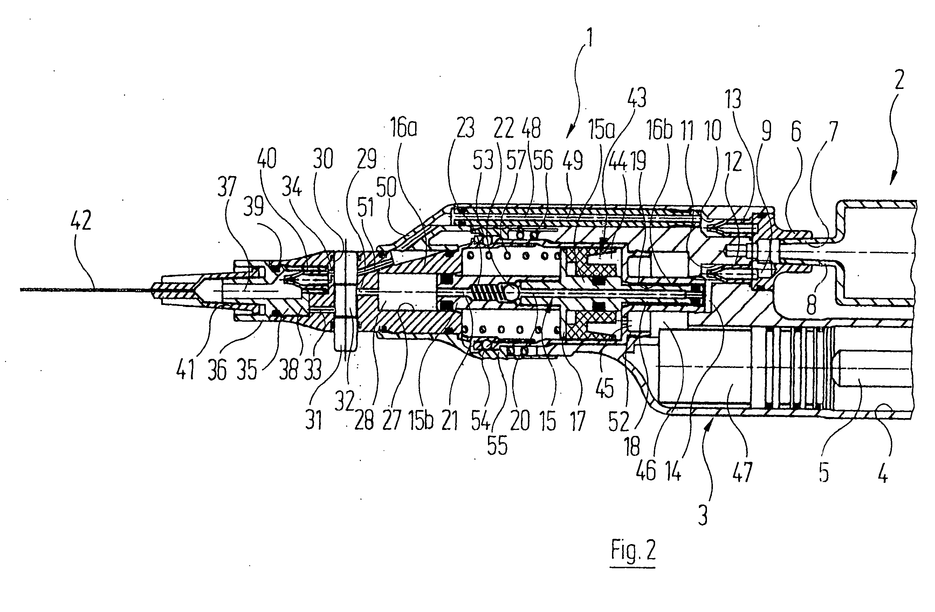

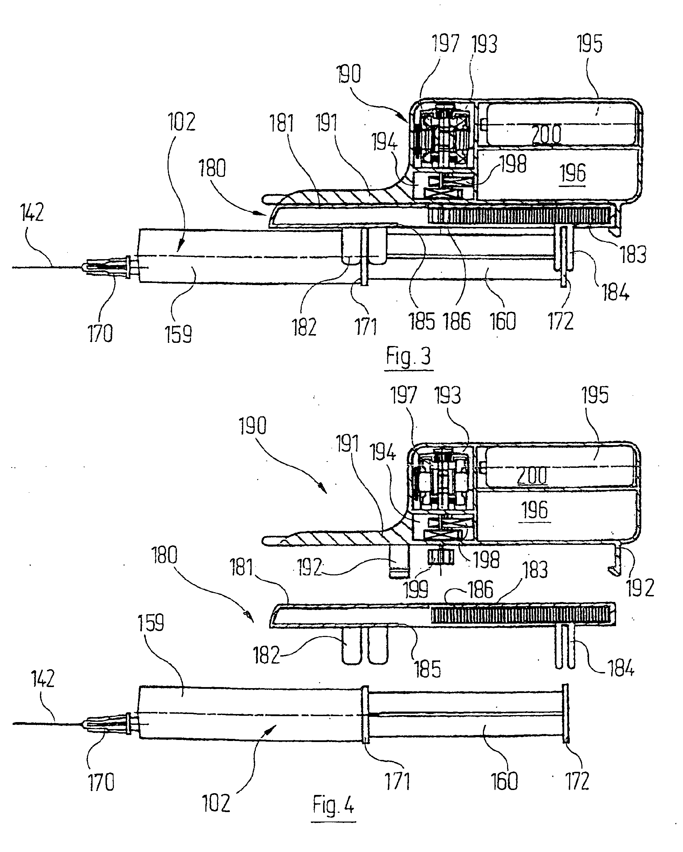

Zunächst wird auf die Figuren 1 und 2 Bezug genommen, in denen ein dentales Behandlungsgerät dargestellt ist, welches mit Druckluft betrieben wird und an den Druckluftanschluß eines herkömmlichen dentalen Handstückes angeschlossen werden kann. Dieses dentale Behandlungsgerät ist somit autark in dem Sinne, daß es keine externen Pumpen und sonstigen Einrichtungen mit Ausnahme des in einer zahnarztpraxis ohnehin vorhandenen Druckluftanschlusses erfordert und mit Hilfe des Fußschalters betätigbar ist, der vom Zahnarzt für die herkömmlichen Handstücke verwendet wird.

Das Behandlungsgerät umfaßt als Hauptkomponente ein Handstück 1, an welches eine bis auf nachfolgende Abweichungen herkömmliche spritze 2 lösbar angesetzt ist.

Wie insbesondere die Figur 2 zeigt, weist das Handstück 1 ein Gehäuse 3 auf, welches auf der in Figur 2 rechten Seite eine Anschlußbohrung 4 für den herkömmlichen Druckluft-Versorgungsschlauch 5 besitzt.

Oberhalb der Anschlußbohrung 4 ist in das Gehäuse 3 ein Anschlußstück 6 eingesetzt, welches eine mittlere Einführungsöffnung 7 für den Hals 8 der Spritze 2 aufweist. Die Einführungsöffnung 7 und die Anschlußbohrung 4 sind im wesentlichen achsparallel.

Die Einführungsöffnung 7 des Anschlußstückes 6 kommuniziert über Räume 9, von denen in der Zeichnung nur einer sichtbar ist, mit zwei achsparallelen Einlaßkanälen 10, 11, in denen jeweils ein Rückschlagventil 12, 13 angeordnet ist. Die Rückschlagventile 12, 13 sind aus elastischem Schlauchmaterial gefertigt, das an dem in der Zeichnung linken, also innenliegenden Ende flachgedrückt ist, derart, daß eine Strömung in der Zeichnung von rechts nach links möglich, eine Strömung in der entgegengesetzten Richtung dagegen unterbunden ist.

Der in den Figuren 1 und 2 untenliegenden Einlaßkanal 11 kommuniziert mit einem ersten Arbeitsraum 14, der zwischen einem linear verschiebbaren, doppelt wirdenden Kolben 15, dem Gehäuse 3 und einem aus zwei Teilen lösbar zusammengefügten Einsatz 16 begrenzt wird.

Auch der Kolben 15 ist aus zwei Teilen 15a, 15b zusammengesetzt. Der in Figur 2 rechte Kolbenteil 15a weist einen radial überstehenden Flansch 17 sowie einen im Durchmesser verringerten Bereich 18 auf, der dicht in einer Bohrung 19 des in den Figuren 1 und 2 rechten Einsatzteiles 16b gleitet. Der gesamte rechte Kolbenteil 15a wird von seiner rechten bis zur linken Stirnseite von einer axialen Bohrung 20 durchsetzt.

Die Bohrung 20 des rechten Kolbenteiles 15a setzt sich in einer axialen Stufenbohrung 21 des in Figur 1 und 2 linken Kolbenteiles 15b fort. In dieser Stufenbohrung 21 ist ein als Kugelventil ausgebildet Rückschlagventil 22 angeordnet, wobei die Kugel dieses Rückschlagventiles 22 durch eine Feder 23 gegen einen ventilsitz gedrückt wird. Die Anordnung ist also so, daß das Rückschlagventil 22 eine Durchströmung der Bohrung 21 des Kolbens 15 in den Figuren 1 und 2 von rechts nach links, nicht jedoch in umgekehrter Richtung zuläßt.

Der linke Endbereich des Kolbens 15 taucht in eine Bohrung 24 des linken Einsatzteiles 16a ein und begrenzt mit dieser einen zweiten Arbeitsraum 28. Der zweite Arbeitsraum 28 steht über eine dünne Axialbohrung 29 mit einer Querbohrung 30 in Verbindung, welche durch das gesamte Einsatzteil 16a hindurchgeführt ist und in welcher ein linear geführter Schieber 31 verschiebbar angeordnet ist. Der Schieber 31 besitzt einen mittleren, im Durchmesser verringerten Bereich 32, so daß dort, so sich dieser mittlere Bereich 32 befindet, zwischen der Querbohrung 30 und dem Schieber 31 ein ringförmiger durchströmbarer Spalt vorliegt.

Von der Querbohrung 30 zweigen zwei schmale, achsparallele Kanäle 33, 34 ab, die in eine Aufnahmeöffnung 35 an der linken Stirnseite des linken Einsatzteiles 16a münden.

In die Aufnahmeöffnung 35 ist ein Kopplungsstück 36 eingesetzt, das eine mittlere, axiale und sich in den Figuren 1 und 2 nach links öffnende Bohrung 37 besitzt. Die Bohrung 37 des Kopplungsstückes 36 kommuniziert über eine schräg geführte Bohrung 38 mit dem unteren Kanal 33 und über eine größere, achsparallel geführte Bohrung 39 mit dem Kanal 34 im linken Einsatzteil 16a. In der Bohrung 39 ist ein Rückschlagventil 40 angeordnet, das in derselben Weise gebaut ist wie die oben schon erörterten Rückschlagventile 12 und 13 und so orientiert ist, daß eine Strömung aus der Bohrung 30 des linken Einsatzteiles 16a in die Bohrung 37 des Kopplungsstückes 36, nicht jedoch in umgekehrter Richtung möglich ist.

Das Kopplungsstück 36 ist an seiner in den Figuren 1 und 2 linken Stirnseite mit den männlichen Anschlußkomponenten einer Luer-Verbindung versehen, welche in die komplementären weiblichen Anschlußkomponenten 41 einer Kanüle 42 eingeführt sind.

In dem Zwischenraum zwischen der Mantelfläche des rechten Kolbenteiles 15a und der Innenmantelfläche des rechten Einsatzteiles 16b ist ein aus elastischem Material gefertigter Betätigungskolben 43 verschiebbar geführt, der mit einer Seite an einer Ringfläche des Flansches 17 des doppeltwirkenden Kolbens 15 anliegt. Ein rechts in den Figuren 1 und 2 von dem Betätigungskolben 43 liegender Druckraum 44 ist durch achsparallel verlaufende Bohrungen 45 mit einem zwischen dem rechten Einsatzteil 16b und dem Gehäuse 3 liegenden Raum 46 in Verbindung. Dieser kommuniziert seinerseits mit dem Auslaß eines handelsüblichen Druckluft-Impulsgebers 47, der am inneren Ende der Einlaßbohrung 4 des Gehäuses 3 angeordnet und in der Lage ist, aus der über die Druckluftleitung 4 zugeführten Druckluft eine Folge von Druckluftimpulsen zu erzeugen, die beispielsweise eine Wiederholfrequenz von 2 Hz aufweist.

Der obere (10) der beiden Einlaßkanäle 10, 11, die der Spritze 2 benachbart sind, setzt sich in einem in diesen Kanal 10 eingeschobenen Drosselteil 48 fort, das von einer Bohrung 49 über die gesamte Länge hinweg durchsetzt wird. Die linke Stirnseite des Drosselteiles 49 kommuniziert über einen Kanal 50 im Gehäuse 3 und einen Kanal 51 im linken Einsatzteil 16a mit der Querbohrung 30, wobei die Mündungsstelle des Kanales 51 in die Querbohrung 30 einen seitlichen Abstand von der Mündungsstelle der Bohrung 29 besitzt, die zum zweiten Arbeitsraum 28 führt.

Der rechte Einsatzteil 16b ist an der Innenwandung des Gehäuses 3 mit Hilfe einer Rastnasen aufweisenden Schürze 52 verrastet.

Die beiden Einsatzteile 16a, 16b sind aneinander durch eine lösbare Schnellkupplung befestigt. Diese Schnellkupplung umfaßt drei in Bohrungen an der Außenmantelfläche des rechten Endbereiches des linken Einsatzteiles 16a einliegende Kugeln 53, die Öffnungen in dem linken Endbereich des rechten Einsatzteiles 16b durchtreten und mit ihrer radial außenliegenden Seite mit der inneren Mantelfläche eines Ringschiebers 55 zusammenwirken. Der Ringschieber 55 wird durch eine Druckfeder 58, die zwischen ihm und einer Stufe des Gehäuses 3 verspannt ist, normalerweise in die in der zeichnung dargestellte Lage gedrückt, in der seine Innenmantelfläche die Kugeln 53 radial nach innen in die Öffnungen des linken Einsatzteiles 16a drückt. Der Ringschieber 55 kann mit Hilfe eines Fingers gegen die Wirkung der Druckfeder 56 in den Figuren 1 und 2 nach rechts bewegt werden, bis die Kugeln 53 vom Ringschieber 55 freikommen. Dann läßt sich das linke Einsatzteil 16a leicht in axialer Richtung entnehmen.

Das oben beschriebene Behandlungsgerät kann in zwei Betriebsarten arbeiten:

In der ersten Betriebsart steht der Schieber 31 so, wie dies in den Figuren 1 und 2 dargestellt ist. In dieser Betriebsart ermöglicht er eine Strömungsverbindung zwischen der Bohrung 29 des linken Gehäuseteiles 16a, welche zum zweiten Arbeitsraum 28 führt, und dem unteren Kanal 33, damit letztendlich zu der Kanüle 42.

Wird nunmehr die Druckluftquelle mit Hilfe des Fußschalters eingeschaltet, so erzeugt der Druckluft-Impulsgeber 47 mit der oben schon erwähnten Wiederholfrequenz von etwa 0,5 bis 2 Hz Druckluftimpulse. Bei jedem dieser Impulse geschieht folgendes:

Die Druckluft, welche über den Raum 46 und die Bohrungen 45 im rechten Einsatzteil 16b in den an den Betätigungskolben angrenzenden Druckraum 44 gelangt, schiebt den Betätigungskolben 43 gegen die Wirkung der Druckfeder 57 in den Figuren 1 und 2 nach links, wobei er aufgrund der Anlage an dem Ringflansch 17 des Kolbens 15 auch letzteren mitnimmt. Bei diesem Hub dringt das in den Figuren 1 und 2 linke Ende des Kolbens 15a tiefer in die Bohrung 27 des linken Einsatzteiles 16a ein und verdrängt auf diese Weise die in dem zweiten Arbeitsraum 28 befindliche Flüssigkeit über die Bohrung 29, den Ringraum zwischen Schieber 31 und Querbohrung 30, den nicht mit einem Rückschlagventil versehenen Kanal 33 und die Bohrung 38 in die Bohrung 37 des Kopplungsstückes 35 und von dort in die Kanüle 42. Die Dimensionen des Arbeitsraumes 28 und der Hub des Kolbens 15 sind dabei so aufeinanderabgestimmt, daß sich ein Auswurfvolumen von etwa 0,3 ml ergibt.

Bei diesem Hub des Kolbens 15 bewegt sich außerdem der im Durchmesser verringerte rechte Endbereich 18 des Kolbens 15 nach links, wobei der erste Arbeitsraum 14 vergrößert wird. Hierdurch wird aus der Spritze 2 über das Rückschlagventil 12 ein bestimmtes Volumen der dort auf Vorrat gehaltenen Behandlungsflüssigkeit angesaugt. Aufgrund des kleineren Durchmessers des Kolbenbereiches 18 ist das angesaugte Flüssigkeitsvolumen geringer als das, welches gleichzeitig aus dem zweiten Arbeitsraum 28 ausgestoßen wird, im Beispiel 0,1 ml.

Sobald der von dem Druckluft-Impulsgeber 47 erzeugte Druckluftimpuls abfällt, werden der Kolben 15 und der Betätigungskolben 43 von der Druckfeder 57 wieder nach rechts in die in den Figuren 1 und 2 dargestellte Position verfahren, wobei der rechts vom Betätigungskolben 43 liegende Druckraum 44 über den Druckluft-Impulsgeber 47 entlüftet wird, Bei dem nach rechts gerichteten Hub des Kolbens 15 geschieht folgendes:

Der linke Endbereich 15b des Kolbens 55 bewegt sich so, daß der zweite Arbeitsraum 28 vergrößert wird und über die Kanüle 42 sowie die oben im einzelnen geschilderte Strömungsverbindung Flüssigkeit in diesen Arbeitsraum 28 zurückgesaugt wird. Aufgrund von Leckverlusten wird dabei jedoch nicht mehr das volle, zuvor ausgeworfene Volumen der Flüssigkeit zurückgesaugt; vielmehr läßt sich über die Kanüle 42 nur noch ein verringertes Flüssigkeitsvolumen von beispielsweise 0,2 ml zurückgewinnen. Während des nach rechts gerichteten Hubes des Kolbens 15 wird jedoch das im ersten Arbeitsraum 14 befindliche Flüssigkeitsvolumen aus diesem Arbeitsraum 14 über die Bohrungen 20, 21 des Kolbens 15 in den zweiten Arbeitsraum 28 verdrängt, wobei sich das Rückschlagventil 22 öffnet. Das im Einlaßkanal 11 befindliche Rückschlagventil 12 bleibt dabei geschlossen. Im zweiten Arbeitsraum 28 befindet sich nunmehr erneut eine Flüssigkeitsmenge von insgesamt etwa 0,3 ml, von denen 0,2 ml über die Kanüle 42 zurückgesaugt und 0,1 ml der Spritze 2 entnommen wurde.

Auf diese Weise verringert sich das Volumen des Flüssigkeitsvorrates in der Spritze 2 kontinuierlich; der Spritzenkolben 58 (vgl. Fig. 1) bewegt sich dabei im Spritzenkörper 59 nach innen, ohne daß auf die Kolbenstange 60 eine Kraft ausgeübt zu werden bräuchte. Dies setzt allerdings voraus, daß der Spritzenkolben 58 sich innerhalb des Spritzenkörpers 59 möglichst reibungsfrei bewegt. Vorzugsweise wird daher als Material für den Spritzenkolben 58 Polytetrafluorethylen eingesetzt.

Sofern Wegwerfspritzen eingesetzt werden, ist eine Kolbenstange 60 nicht erforderlich, da der Spritzenkolben 58 nur dem Abschluß des sich in seinem volumen ändernden Innenraumes des Spritzenkörpers 59 dient. Eine Kolbenstange 60 wird nur bei wiederverwendbaren Spritzen 2 zum Wiederbefüllen des Spritzenkörpers 59 benötigt.

Das beschriebene Behandlungsgerät kann in einer zweiten Betriebsart benutzt werden, die dem Spülen dient, ohne daß eine Rücksaugung von Behandlungsflüssigkeit über die Kanüle 42 erfolgt. Hierzu wird der Schieber 31 in der Querbohrung 30 des linken Gehäuseeinsatzes 16 so verschoben, daß er eine Verbindung zwischen der Bohrung 29, dem Kanal 51 und dem Kanal 34 des linken Einsatzteiles 16a schafft-Wird in dieser Stellung des Schiebers 31 die Druckluftquelle eingeschaltet, so geschieht folgendes:

Bei jedem nach links gerichteten Hub des Kolbens 15 wird, wie zuvor geschildert, der gesamte Inhalt von etwa 0,3 ml des zweiten Arbeitsraumes 28 ausgestoßen und über den Kanal 34 sowie das Rückschlagventil 40 zur Kanüle 42 befördert. Beim in entgegengesetzter Richtung verlaufenden Hub des Kolbens 15 jedoch ist ein Rücksaugen von Flüssigkeit aus der Kanüle 42 wegen des sich schließenden Rückschlagventiles 40 nicht möglich. Zwar wird in gleicher Weise wie in der ersten Betriebsart das im ersten Arbeitsraum 14 befindliche, aus der Spritze 2 zuvor angesaugte Flüssigkeitsvolumen in den zweiten Arbeitsraum 28 verdrängt; die restliche zur vollständigen Füllung des zweiten Arbeitsraumes 28 erforderliche Menge an Behandlungsflüssigkeit, im Beispiel 0,2 ml, werden über die Bohrung 51 des linken Einsatzteiles 16a, die Bohrung 50 des Gehäuses 3, die Bohrung 49 des Drosselteiles 48 und das Rückschlagventil 13 ebenfalls aus der Spritze 2 angesaugt. Dies bedeutet, daß über die Kanüle 42 stets nur frische Flüssigkeit austritt, womit eine Spülung bewirkt werden kann.

## Patentansprüche

1. Dentales Behandlungsgerät zum Infiltrieren und/oder spülen von Gewebe oder gewebebegrenzten Hohlräumen, insbesondere von Zahngewebe oder zahngewebe-begrenzten Hohlräumen, mit einer Behandlutngsflüssigkeit mit
a) einem vorratsbehälter für die Behandlungsflüssigkeit;
b) einer Kanüle zum Einbringen der Behandlungsflüssigkeit in das Gewebe bzw. die Hohlräume;
c) einer Pumpe, welche der Kanüle die Behandlungsflüssigkeit aus dem Vorratsbehälter zuführt;
d) einer Pumpe, welche über die Kanüle Behandlungsflüssigkeit aus dem Gewebe absaugt,
e) bei der Vorratsbehälter (2; 102), Kanüle (42; 142) und Pumpen (15, 43, 47; 160, 180, 191) zu einer handstückartigen Einheit zusammengefaßt sind,
f) und bei der die Pumpe, welche die Behandlungsflüssigkeit der Kanüle (42; 142) zuführt, und die Pumpe, welche die Behandlungsflüssigkeit über die Kanüle (42; 142) ansaugt, durch eine einzige Pumpe (15, 43, 47; 160, 180, 191) implementiert sind, deren Arbeitsrichtung umkehrbar ist,
**dadurch gekennzeichnet, daß**
g) die einzige Pumpe einen doppelt wirkenden, linear beweglichen Kolben (15) umfaßt, der mit einem Endbereich (18) an einen ersten Arbeitsraum (14) angrenzt, welcher über ein Rückschlagventil (12) mit dem Reservoir (2) verbunden ist, und mit dem gegenüberliegenden Endbereich (15b) an einen zweiten Arbeitsraum (28) angrenzt, der mit der Kanüle (42) kommuniziert, wobei der erste Arbeitsraum (14) mit dem zweiten Arbeitsraum (28) über einen Strömungsweg (20, 21) kommuniziert, in welchem ein Rückschlagventil (22) liegt, das eine Strömung der Behandlungsflüssigkeit nur vom ersten Arbeitsraum (14) in den zweiten Arbeitsraum (28) zuläßt.

2. Behandlungsgerät nach Anspuch 1, **dadurch gekennzeichnet, daß** der vom ersten Arbeitsraum (14) zum zweiten Arbeitsraum (28) führende Strömungsweg eine axial durch den Kolben (15) geführte Bohrung (20, 21) ist.

3. Behandlungsgerät nach Anspruch 1 oder 2, **dadurch gekennzeichnet, daß** der Querschnitt des an den ersten Arbeitsraum (14) angrenzenden Endbereichs (18) des Kolbens (15) kleiner ist als der Querschnitt des an den zweiten Arbeitsraum (28) angrenzenden Endbereiches (15b) des Kolbens (15).

4. Behandlungsgerät nach einem der Ansprüche 1 bis 3, **dadurch gekennzeichnet, daß** ein Steuerventil (31) vorgesehen ist, welches in einer ersten Stellung den zweiten Arbeitsraum (28) über einen in beiden Richtungen durchströmbaren Strömungsweg (33, 37) mit der Kanüle (42) verbindet und in einer zweiten Stellung den zweiten Arbeitsraum (28) über einen nur in Richtung zur Kanüle (42) durchströmbaren Strömungsweg (34, 39, 40) mit der Kanüle (42) und mit einem weiteren, zum Reservoir (2) führenden Strömungsweg (10, 49, 50, 51) verbindet, wobei in dem weiteren Strömungsweg (10, 49, 50) ein Rückschlagventil (13) liegt, das ausschließlich eine Strömung in Richtung zum zweiten Arbeitsraum (28) zuläßt.

5. Behandlungsgerät nach Anspruch 4, **dadurch gekennzeichnet, daß** das steuerventil einen in einer Bohrung (30) linear verschiebbaren Schieber (31) umfaßt.

6. Behandlungsgerät nach einem der Ansprüche 1 bis 5, **dadurch gekennzeichnet, daß** der doppelt wirkende Kolben (15) von einem Betätigungskolben (43) angetrieben ist, der an einer Seite durch eine Druckfeder (57) beaufschlagt ist und an der gegenüberliegenden Seite an einen Druckraum (44) angrenzt, der seinerseits mit dem Ausgang eines Druckluft-Impulsgebers (47) kommuniziert.

7. Behandlungsgerät nach Anspruch 6, **dadurch gekennzeichnet, daß** der Eingang des Druckluft-Impulsgebers (47) über eine Standardkupplung mit einem Druckluft-Versorgungskabel (5) für übliche zahnärztliche Handstücke verbindbar ist.

8. Behandlungsgerät nach einem der Ansprüche 1 bis 3, **dadurch gekennzeichnet, daß** das Reservoir (2) eine lösbar angebrachte Spritze ist, die einen leichtgängigen Spritzenkolben (58) aufweist.

9. Behandlungsgerät nach Anspruch 8, **dadurch gekennzeichnet, daß** die Spritze (2) eine wiederverwertbare, aus autoklavierbarem Material bestehende Spritze ist.

10. Behandlungsgerät nach Anspruch 8, **dadurch gekennzeichnet, daß** die Spritze (2) eine Wegwerfspritze ist.

11. Behandlungsgerät nach Anspruch 10, **dadurch gekennzeichnet, daß** die Wegwerfspritze (2) keine Kolbenstange aufweist.

## Claims

1. A dental therapeutic instrument for infiltrating and/or rinsing tissue or cavities bounded by tissue, in particular dental tissue or cavities bounded by dental tissue, with a therapeutic liquid comprising
a) a storage container for the therapeutic liquid;
b) a cannula for introducing the therapeutic liquid into the tissue or into the cavities;
c) a pump which supplies the therapeutic liquid to the cannula from the storage container;
d) a pump which withdraws therapeutic liquid from the tissue by suction via the cannula;
e) wherein the storage container (2; 102), the cannula (42; 142) and the pumps (15, 43, 47; 160, 180, 191) are combined into a handpiece-type unit;
f) and wherein the pump that supplies the therapeutic liquid to the cannula (42; 142) and the pump that aspirates the therapeutic liquid via the cannula (42; 142) are implemented by a single pump (15, 43, 47; 160, 180, 191), the working direction of which is reversible;
g) the single pump comprises a double-acting, linearly mobile piston (15) which with one end region (18) borders a first working space (14) which is connected to the storage container (2) via a check valve (12) and with the opposite end region (15b) borders a second working space (28) which communicates with the cannula (42), the first working space (14) communicating with the second working space (28) via a flow path (20, 21) in which a check valve (22) is situated which permits a flow of the therapeutic liquid only from the first working space (14) into the second working space (28).

2. Therapeutic instrument according to Claim 1, **characterized in that** the flow path leading from the first working space (14) to the second working space (28) is a bore (20, 21) which is directed axially through the piston (15).

3. Therapeutic instrument according to Claim 1 or 2, **characterized in that** the cross-section of the end region (18) of the piston (15) adjoining the first working space (14) is smaller than the cross-section of the end region (15b) of the piston (15) adjoining the second working space (28).

4. Therapeutic instrument according to one of Claims 1 to 3, **characterized in that** a control valve (31) is provided which in a first position connects the second working space (28) to the cannula via a flow path (33, 37) that is capable of being flowed through in both directions and in a second position connects the second working space (28) to the cannula (42) and to a further flow path (10, 49, 50, 51) leading to the storage container (2) via a flow path (34, 39, 40) that is capable of being flowed through only in the direction towards the cannula (42), a check valve (13) which exclusively permits a flow in the direction towards the second working space (28) being situated in the further flow path (10, 49, 50).

5. Therapeutic instrument according to Claim 4, **characterized in that** the control valve comprises a slide (31) which is capable of being displaced linearly in a bore (30).

6. Therapeutic instrument according to one of Claims 1 to 5, **characterized in that** the double-acting piston (15) is driven by an actuating piston (43) which is acted upon on one side by a compression spring (57) and which on the opposite side adjoins a pressure chamber (44) which in turn communicates with the outlet of a compressed-air pulse generator (47).

7. Therapeutic instrument according to Claim 6, **characterized in that** the inlet of the compressed-air pulse generator (47) is capable of being connected to a compressed-air supply cable (5) for conventional dental handpieces via a standard coupling.

8. Therapeutic instrument according to one of Claims 1 to 3, **characterized in that** the reservoir (2) is a detachably fitted syringe which exhibits a smooth-running syringe piston (58).

9. Therapeutic instrument according to Claim 8, **characterized in that** the syringe (2) is a re-usable syringe consisting of autoclavable material.

10. Therapeutic instrument according to Claim 8, **characterized in that** the syringe (2) is a disposable syringe.

11. Therapeutic instrument according to Claim 10, **characterized in that** the disposable syringe (2) has no piston rod.

## Revendications

1. Appareil de traitement dentaire pour infiltrer et ou rincer avec un liquide de traitement des tissus ou des cavités délimitées par des tissus, en particulier des tissus dentaires ou des cavités délimitées par des tissus dentaires, comprenant
a) un réservoir pour le liquide de traitement ;
b) une canule pour introduire le liquide de traitement dans le tissu ou les cavités ;
c) une pompe qui apporte à la canule le liquide de traitement provenant du réservoir ;
d) une pompe qui, par l'intermédiaire de la canule, aspire le liquide de traitement hors du tissu ;
e) selon lequel le réservoir (2 ; 102), la canule (42 ; 142) et les pompes (14, 43, 47 ; 160, 180, 191) sont réunis en une unité du genre pièce à main,
f) et selon lequel la pompe qui apporte le liquide de traitement à la canule (42 ; 142) et la pompe qui aspire le liquide de traitement par l'intermédiaire de la canule (42 ; 142) sont mises en oeuvre sous la forme d'une unique pompe (14, 43, 47 ; 160, 180, 191) dont le sens de travail est réversible,
**caractérisé en ce que**
g) l'unique pompe comprend un piston (15) à déplacement linéaire à double action, qui est limitrophe par une région terminale (18) d'une première chambre de travail (14) qui est reliée au réservoir (2) par l'intermédiaire d'un clapet anti-retour (12), et est limitrophe par la région terminale opposée (15b) d'une deuxième chambre de travail (28) qui communique avec la canule (42), sachant que la première chambre de travail (14) communique avec la deuxième chambre de travail (28) par l'intermédiaire d'un chemin d'écoulement (20, 21) dans lequel se trouve un clapet anti-retour (22) qui n'autorise un écoulement du liquide de traitement que de la première chambre de travail (14) dans la deuxième chambre de travail (28).

2. Appareil de traitement selon la revendication 1, **caractérisé en ce que** le chemin d'écoulement menant de la première chambre de travail (14) dans la deuxième chambre de travail (28) est un perçage (20, 21) dirigé axialement à travers le piston (15).

3. Appareil de traitement selon la revendication 1 ou 2, **caractérisé en ce que** la section de la région terminale (18) du piston (15) qui est limitrophe de la première chambre de travail (14) est plus petite que la section de la région terminale (15b) du piston (15) qui est limitrophe de la deuxième chambre de travail (28).

4. Appareil de traitement selon l'une des revendications 1 à 3, **caractérisé en ce qu'**il est prévu une soupape de commande (31) qui, dans une première position, relie la deuxième chambre de travail (28) à la canule (42) par l'intermédiaire d'un chemin d'écoulement (33, 37) pouvant être parcouru par un flux dans les deux sens, et qui, dans une deuxième direction, relie la deuxième chambre de travail (28), par l'intermédiaire d'un chemin d'écoulement (34, 39, 40) ne pouvant être parcouru par un flux qu'en direction de la canule (42), à la canule (42) et à un autre chemin d'écoulement (10, 49, 50, 51) menant au réservoir (2), sachant qu'un clapet anti-retour (13) qui n'autorise qu'un écoulement en direction de la deuxième chambre de travail (28) se trouve dans l'autre chemin d'écoulement (10, 49, 50).

5. Appareil de traitement selon la revendication 4, **caractérisé en ce que** la soupape de commande comprend un tiroir (31) pouvant être déplacé linéairement dans un perçage (30).

6. Appareil de traitement selon l'une des revendications 1 à 5, **caractérisé en ce que** le piston (15) à double action est entraîné par un piston d'actionnement (42) qui est sollicité sur un côté par un ressort de compression (57) et qui, sur le côté opposé, est limitrophe d'une chambre de pression (44) qui communique elle-même avec la sortie d'un générateur (47) d'impulsions d'air comprimé.

7. Appareil de traitement selon la revendication 6, **caractérisé en ce que** l'entrée du générateur (47) d'impulsions d'air comprimé peut être reliée par l'intermédiaire d'un accouplement standard à un câble (5) d'alimentation en air comprimé pour des pièces à main dentaires courantes.

8. Appareil de traitement selon l'une des revendications 1 à 3, **caractérisé en ce que** le réservoir (2) est une seringue montée de manière amovible, qui présente un piston de seringue (58) fonctionnant sans effort.

9. Appareil de traitement selon la revendication 8, **caractérisé en ce que** la seringue (2) est une seringue réutilisable constituée d'un matériau pouvant être passé à l'autoclave.

10. Appareil de traitement selon la revendication 8, **caractérisé en ce que** la seringue (2) est une seringue à usage unique.

11. Appareil de traitement selon la revendication 10, **caractérisé en ce que** la seringue à usage unique (2) ne présente pas de tige de piston.
